# FASCICULE DE BREVET EUROPEEN

(11) **EP 0 129 076 B1**
(45) Date de publication et mention de la délivrance du brevet: **24.03.1993**
(21) Numéro de dépôt: 84105677.3
(22) Date de dépôt: 18.05.1984
(51) Int. Cl.: G01N 33/36

(54) **Appareil de contrôle automatique de fils textiles et procédé mettant en oeuvre cet appareil**
Einrichtung zur automatischen Kontrolle von Textilfäden und Methode zur Anwendung dieser Einrichtung
Apparatus for quality control of textile threads, and method based on the use of said apparatus

(30) Priorité: 21.06.1983 FR 8310360
(43) Date de publication de la demande: 27.12.1984
(73) Titulaire: SUPERBA S.A., 68060 Mulhouse (FR)
(72) Inventeur: Enderlin, Robert, F-68790 Morschwiller (FR); Schutz, Richard Adrien, F-68200 Mulhouse (FR); Drean, Jean-Yves, F-68200 Brunstatt (FR)
(74) Mandataire: Nithardt, Roland

(56) Documents cités:
- DE-A- 2 363 771
- DE-A- 2 745 043
- FR-A- 2 244 982
- GB-A- 888 576
- US-A- 3 751 981
- US-A- 3 788 138
- US-A- 4 116 393
- "Sonderdruck aus MELLIAND TEXTILBERICHTE", vol. 37, Heidelberg (DE), April 1956; H.STEIN, pp. 1-7*
- "Sonderdruck aus REYON, ZELLWOLLE UND ANDERE CHEMIEFASERN", Heft 9-12/1956; H.STEIN, pp. 1-15*

## Description

La présente invention concerne un appareil de contrôle automatique de fils textiles ainsi qu'un procédé mettant en oeuvre cet appareil.

Le contrôle des fils devient de plus en plus nécessaire, surtout depuis la production de filés fabriqués selon différents modes de filature tels que la filature classique sur continu à anneau et curseur, ou la filature à fibres libérées et leurs différentes variantes. En effet, l'aspect, le comportement aux sollicitations mécaniques et même le comportement à la teinture de fils réalisés selon ces différents modes de filature, même à partir de fibres identiques présentent des différences hautement significatives qui font que l'utilisation de ces fils ne peut être que spécifique. En d'autres termes, des fils même réalisés à partir des mêmes matières premières mais obtenus selon des procédés de filature différents, conduiront, lorsqu'ils sont assemblés dans le même tissu ou le même tricot, à des défauts d'aspect se présentant sous la forme de barres, d'irrégularités rédhibitoires, etc... De plus, il est fréquent que le fabricant d'étoffes ne produise pas lui-même tous les fils qu'il utilise.

D'une manière générale, les filés fabriqués selon le mode classique sur continus à anneaux, sont suffisamment proches les uns des autres du point de vue structure, de sorte que le plus souvent, un contrôle simple de titre et de régularité de masse linéique est suffisant pour prévenir les accidents de fabrication au tissage et/ou en bonneterie, bien que des différences de torsion peuvent, si elles sont suffisamment importantes, être également à l'origine de certains défauts.

A l'heure actuelle, avec un marché de filés réalisés selon des modes fort variés, le contrôle du titre et de la régularité de ce titre c'est-à-dire de la régularité de masse linéique, devient insuffisant pour prévenir les défauts. De plus, à torsion moyenne nominale égale, ainsi qu'à titre et à régularité moyenne égaux, les filés obtenus par filature à fibres libérées sont différents en structure des filés obtenus de manière classique. Mais, même des filés comportant ces mêmes caractéristiques globales, obtenus sur différentes machines de filature à fibres libérées, ou encore sur les mêmes machines mais à réglages, à degrés d'usure ou de propreté différents, peuvent présenter des structures suffisamment différenciées pour être à l'origine des défauts susmentionnés apparents sur les étoffes.

Des études relativement récentes, décrites notamment dans la publication ayant pour titre "De la torsion des filés : notion, détermination, application à la caractérisation de la structure et de l'aspect" de R.A. SCHUTZ et de J.Y DREAN, parue dans "l'Industrie Textile" n° 1089 du mois de mai 1979, pages 421-427, dans la publication "Charakterisierung des Garne, hinsichtlich ihrer Verarbeitung und Eigenschaften" des mêmes auteurs, parue dans "Melliand Textiberichte" du mois d'août 1979, pages 656-659 et dans la publication ayant pour titre "Charakterisierung der Garne" de R.A. SCHUTZ, J.Y. DREAN, D. CARRIERE, parue dans "Textilsveredlung" du mois de janvier 1981, pages 9-11, ont conduit à de nouvelles méthodes de caractérisation des filés concernant notamment les filés obtenus en filature à fibres libérées et permettant de distinguer en valeur chiffrée les différentes structures de ces fils.

Ces méthodes fiables, éprouvés par des essais interlaboratoires, présentent toutefois l'inconvénient d'être relativement longues et fastidieuses. En effet, jusqu'à ce jour et pour les raisons évoquées plus haut, les différents contrôles sur filés sont effectués à l'aide d'appareils spécifiques, notamment un dévidoir et une balance pour la détermination du titre, un régularimètre pour la détermination de la régularité, un torsiomètre pour la détermination de la torsion et un dynamomètre pour la détermination des caractéristiques d'allongement et de résistance à la rupture.

On connait déjà divers appareils de mesure des caractéristiques de fils textiles, et notamment ceux décrits dans les articles rédactionnels de Herbert Stein parus dans le Melliand Textilberichte, numéro 37 d'avril 1956 (pages 467 à 473) et dans le Reyon, Zellwolle und andere Chemiefasern, numéro 9 de décembre 1956 (pages 1 à 16). Il s'agit en fait d'appareils rudimentaires, monofonctionnels, sans aucun automatisme et non interactifs.

Parmi les différents types d'appareils disponibles sur le marché, certains ont été plus ou moins automatisés mais restent spécifiques au contrôle qu'ils permettent d'effectuer. Pourtant, compte tenu des normes et des prescriptions liées aux méthodes utilisées, il est nécessaire de connaître a priori certaines caractéristiques obtenues sur d'autres appareils pour effectuer certaines de ces mesures. Ainsi la tension, préalable pour plusieurs types de mesures (torsion, dynamométrie, etc...) est à appliquer en fonction du titre du filé qu'il faut connaître ou déterminer préalablement.

Compte tenu du fait que, d'une part un type de détermination unique s'avère de plus en plus insuffisant pour la caractérisation des filés, que d'autre part, certaines déterminations sont longues et nécessitent l'action et/ou l'intervention d'un opérateur, il apparait important de repenser le contrôle sur une autre base, c'est-à-dire selon une méthodologie et un système de mesure appropriés.

La présente invention se propose de pallier les inconvénients susmentionnés et de mettre à disposition de l'industrie textile un procédé de contrôle automatique des fils textiles ainsi qu'un appareil simple et efficace permettant de mettre en oeuvre ce procédé pour effectuer un ensemble de mesures permettant une caractérisation précise des filés quel que soit leur mode d'obtention.

Dans ce but, l'appareil de contrôle automatique d'un fil textile selon l'invention est caractérisé en ce qu'il comporte un caisson contenant d'une part deux modules de mesure pourvus, l'un, de moyens pour effectuer des mesures sur ce fil permettant de déterminer son titre et sa régularité de masse linéique, l'autre, de moyens pour effectuer des mesures sur ce fil permettant de déterminer sa torsion et ses propriétés dynamométriques, et d'autre part un manipulateur et une pince pour prélever des éprouvettes de fil d'une position d'attente et pour les amener vers ledit premier ou ledit deuxième module, et en ce qu'il comporte en outre des moyens utilisant le résultat de la détermination du titre pour calculer et appliquer automatiquement une tension préalable au fil lors des mesures permettant la détermination de la torsion et des propriétés dynamométriques, et des moyens pour réitérer automatiquement ces mesures sur une serie d'éprouvettes d'un même fil, ainsi que des moyens pour enregistrer en mémoire les résultats de ces mesures et des moyens informatiques pour traiter ces résultats en vue de leur exploitation statistique et de leur archivage.

Selon un mode de réalisation avantageux, le premier module comporte un organe de mesure, par voie capacitive, de la régularité du titre du fil, un organe de mesure du titre de ce fil, et un dispositif d'entraînement, ces trois composants étant disposés sur une même verticale et agencés pour coopérer à la détermination simultanée du titre d'un fil et de la régularité de ce titre.

L'organe de mesure de la régularité du titre comporte avantageusement un condensateur double comprenant une plaque centrale et deux plaques latérales, définissant deux couloirs dont l'un est traversé par un fil à tester, ainsi qu'un circuit électronique de commande comportant un oscillateur agencé pour appliquer sur les plaques latérales une tension alternative en opposition de phase, un amplificateur et un démodulateur pour détecter les variations de tension sur la plaque centrale et un circuit de contre-réaction comprenant un convertisseur analogique-numérique et un convertisseur numérique-analogique pour masquer les déséquilibres du pont capacitif comprenant les plaques du condensateur double, le démodulateur étant connecté à une unité centrale de traitement des données, en vue du traitement statistique et de l'archivage des résultats des mesures.

Le dispositif d'entrainement associé aux deux organes de mesure du premier module, comporte de préférence un berceau fixé à la plaque de montage, un rouleau d'entrainement couplé à un moteur d'entrainement pas-à-pas et un rouleau presseur pourvu d'un revêtement antidérapant, ce rouleau presseur étant pourvu de moyens pour assurer une pression constante de sa surface périphérique contre la surface périphérique du rouleau d'entrainement.

Pour faciliter l'insertion automatique d'un fil entre le rouleau presseur et le rouleau d'entraînement, le rouleau presseur comporte le long de son rebord périphérique extérieur au moins une entaille, et de préférence une série d'entailles juxtaposées.

L'organe de mesure du titre comporte, selon un mode de réalisation préféré, une balance de précision posée sur une console solidaire de la plaque de montage, équipée d'un réceptacle pour recevoir des longueurs contrôlées de fil, et d'une sortie des signaux correspondant aux mesures successives, ces signaux étant transmis à l'unité centrale de traitement des données en vue de l'exploitation statistiques et de l'archivage des résultats des mesures.

Le second module conçu pour déterminer la torsion et les propriétés dynamométriques d'une éprouvette de fil comporte un dispositif comprenant un moteur pas-à-pas portant une pince entrainée en rotation dans un sens ou dans l'autre par ce moteur, et agencé pour retenir une extrémité d'une éprouvette de fil à tester, un dispositif comprenant une pince et un capteur de force, et des moyens mécaniques pour déplacer le dispositif en hauteur le long d'une fente verticale ménagée dans la plaque de montage.

Les moyens mécaniques pour déplacer ce dispositif en hauteur comportent un moteur pas-à-pas et une vis verticale entrainée en rotation par ce moteur, cette vis étant couplée à ce dispositif pour assurer son déplacement vertical.

Afin d'éviter l'encrassement de l'appareil par des restes de fil ou les éprouvettes de fil après les mesures, l'appareil comporte avantageusement des moyens pour aspirer automatiquement ces résidus.

Par ailleurs, pour permettre le traitement simultané de deux éprouvettes dans les deux modules de mesure de l'appareil, celui-ci comporte de préférence un déflecteur conçu pour permettre de définir deux zones séparées, dont l'une contient une éprouvette de fil testée par le premier module et dont l'autre contient une éprouvette de fil testée par le second module.

La mise en place des fils pour qu'ils soient mesurés dans l'un ou l'autre des modules de mesure, s'effectue au moyen du manipulateur commandé par l'unité de traitement central des données, et qui comprend un disque rotatif, un bloc de guidage monté sur deux rails fixés perpendiculairement à ce disque de telle manière que ce bloc puisse coulisser le long de ces rails, une tige coulissant à l'intérieur du bloc de guidage, un bras de manipulation monté à l'extrémité libre de la tige et portant la pince pour prélever des fils de leurs positions d'attente et pour les amener vers un organe de mesure ou pour couper une longueur prédéterminée de ces fils.

Enfin, cet appareil comporte avantageusement un râtelier, monté de préférence à l'extérieur du caisson contenant les instruments de mesure, ce râtelier étant formé d'un châssis portant un ensemble de tiges agencées pour recevoir des bobines de fil à tester, des tubes d'aspiration pour aspirer ces fils et pour les amener au travers de tubes flexibles à l'intérieur de l'appareil de contrôle.

Dans ce but, également le procédé de contrôle automatique d'un fil textile selon l'invention est caractérisé en ce que ces mesures permettent de déterminer le titre de ce fil, sa régularité de masse linéique, sa torsion et ses propriétés dynamométriques, en ce que l'on utilise le résultat de la détermination du titre pour calculer et appliquer automatiquement une tension préalable au fil lors des mesures permettant la détermination de la torsion et des propriétés dynamométriques, en ce que l'on réitère automatiquement les mesures et qu'on enregistre en mémoire les résultats de ces mesures pour les traiter par voie informatique en vue de leur exploitation statistique et de leur archivage.

De préférence, on effectue les mesures pour déterminer le titre et la régularité de masse linéique au moyen d'une première éprouvette dudit fil et en ce que l'on effectue les mesures pour déterminer la torsion et les caractéristiques dynamométriques au moyen d'une deuxième éprouvette de ce fil.

Pour effectuer ces mesures, on introduit de préférence une série d'éprouvettes de fil dans l'appareil de telle manière que leurs extrémités soient localisées chacune dans une position d'attente d'où elles peuvent être reprises pour être introduites successivement dans un des modules de mesure, en ce que l'on effectue successivement pour chaque fil une série de mesures répétées au moyen des organes de mesures d'un même module, et en ce que l'on arrête cette série de mesures soit lorsqu'un nombre prédéterminé de mesures a été effectué, soit lorsque la précision statistique est atteinte pour un nombre de mesures inférieur audit nombre prédéterminé.

La mise en place des éprouvettes de fil préalablement à la phase de mesure proprement dite s'effectue de préférence, en partie automatiquement à l'aide de jets d'air pour introduire les éprouvettes dans l'appareil de contrôle, et en partie manuellement pour fixer les extrémités libres de ces fils dans leurs positions d'attente respectives à l'intérieur de l'appareil.

Grâce à une disposition particulière des organes de mesure, les éprouvettes sont positionnées verticalement au cours des mesures effectuées dans chacun des modules.

En outre, l'on évacue automatiquement, par aspiration, les déchets ainsi que les éprouvettes ayant fait l'objet de mesures.

La présente invention sera mieux comprise en référence à la description d'un exemple de réalisation et du dessin annexé dans lequel:
La figure 1 représente une vue schématique en perspective de l'appareil de contrôle automatique selon l'invention,
La figure 2 représente une vue schématique en perspective du râtelier portant une série de bobines de fils à contrôler,
La figure 3 représente une vue en perspective des pinces de fixation des éprouvettes de fil dans leur position d'attente a l'intérieur de l'apareil représenté par la fig. 1,
La figure 4 représente une vue schématique de l'organe de mesure de la régularité de masse linéique monté dans l'appareil de la fig. 1,
La figure 5 illustre sous forme de schéma-bloc le circuit associé à l'organe de mesure représenté par la fig. 4,
La figure 6 représente une vue en élévation de la balance de précision de l'organe de mesure permettant la détermination du titre,
La figure 7 est une vue en élévation du dispositif d'entrainement du premier module de mesure,
La figure 8 représente une vue en coupe transversale des rouleaux d'entrainement illustrés par la fig. 7, et
La figure 9 représente une vue en coupe de l'organe de mesure des propriétés dynamométriques, monté dans le second module de l'appareil de la fig. 1.

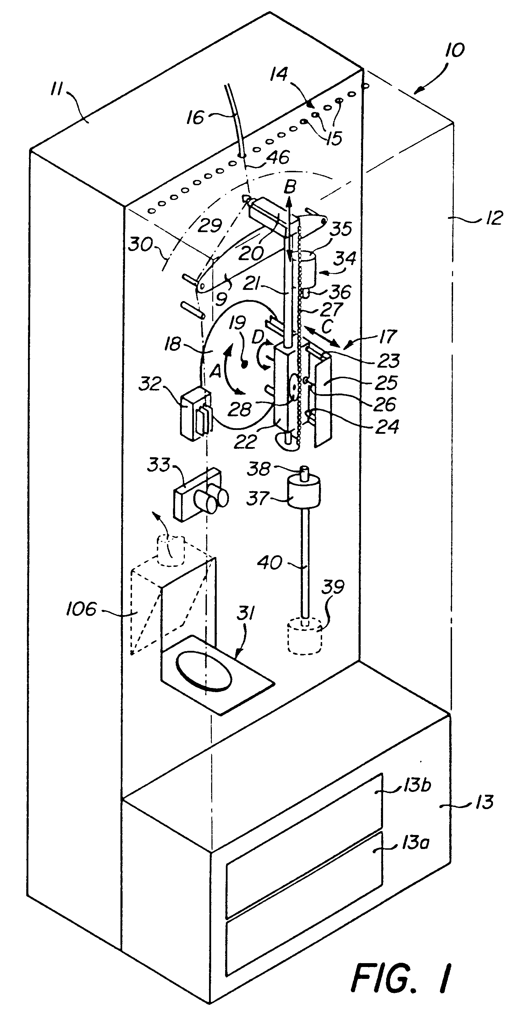

En référence aux figures et notamment à la fig. 1, l'appareil représenté se compose d'un caisson 10 contenant une plaque de montage 11 verticale, servant de support à l'ensemble des instruments de mesure, une porte frontale 12, de préférence transparente, qui permet à l'opérateur d'accéder aux instruments de mesure, une base 13 qui contient les organes de commande 13a des moyens d'entrainement et l'unité centrale 13b de traitement des données. Une console (non représentée) équipée d'un clavier pour introduire les données et d'un écran cathodique, connus en soi, est associée à l'appareil représenté par la fig. 1. Le caisson 10 est de préférence réalisé à l'aide d'un châssis rigide habillé par des plaques en tôle ou en matière synthétique, montées sur ce châssis. Toutefois, cette réalisation peut être modifiée en fonction des utilisations prévues ou au choix de l'utilisateur ou du constructeur.

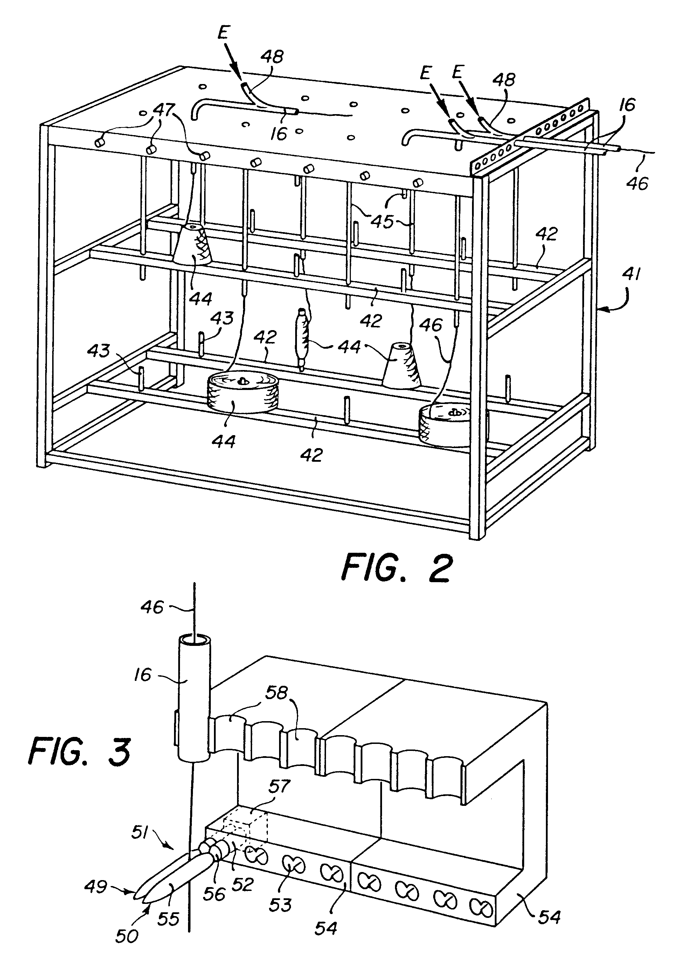

La plaque supérieure 14 du caisson 10 comporte une série d'orifices 15 dans lesquels sont fixés des tubes flexibles 16 destinés à amener les éprouvettes de fil à l'intérieur de l'appareil. Ces éprouvettes proviennent du râtelier (représenté par la fig. 2) d'une manière qui sera décrite plus en détail par la suite. Elles pénètrent par les tubes 16 et leurs extrémités sont coupées manuellement et mises en place dans des pinces appropriées décrites en référence à la fig. 3.

La plaque de montage 11 verticale, porte un manipulateur 17 mobile selon trois axes orthogonaux, et à commande informatisée. Ce manipulateur 17 comporte d'une manière connue en soi un disque 18 rotatif, dans le sens de la double flèche A autour d'un axe 19 porté par un palier (non représenté) et monté sur la face arrière de la plaque 11, et un bras de manipulation 20 fixé à angle droit à l'extrémité libre d'une tige 21 mobile dans le sens de la double flèche B. Cette tige 21 est portée par un bloc de guidage 22, lui-même supporté par deux rails 23 et 24 montés entre le disque 18 et un support coudé 25 également porté par ce disque. L'entrainement en rotation du disque 18 se fait par un moteur d'entrainement pas-à-pas (non représenté) monté sur la face arrière de la plaque 11. Le déplacement du bloc de guidage 22 dans le sens de la double flèche C au moyen d'une vis 26 entrainée dans le sens de la double flèche D, s'effectue au moyen d'un moteur pas-à-pas (non représenté). Le déplacement de la tige 21 est obtenu grâce à une chaîne 27 tendue entre les deux extrémités de cette tige et qui engrène avec un pignon denté 28 entrainé en rotation dans un sens ou dans le sens oppposé par un moteur pas-à-pas (non représenté) logé dans ou porté par le bloc de guidage 22. L'extrémité libre du bras de manipulation 20 porte une pince 29 dont la fonction consiste à pincer et/ou à couper les éprouvettes de fil à tester. Cette pince, ne faisant pas partie de l'objet de la présente invention n'est pas décrite plus en détail dans ce texte.

Le rôle du manipulateur 17 consiste à prélever les extrémités de fils préalablement mises en place dans leurs positions d'attente dans les pinces représentées par la fig. 3 et disposées selon un arc de cercle 30.

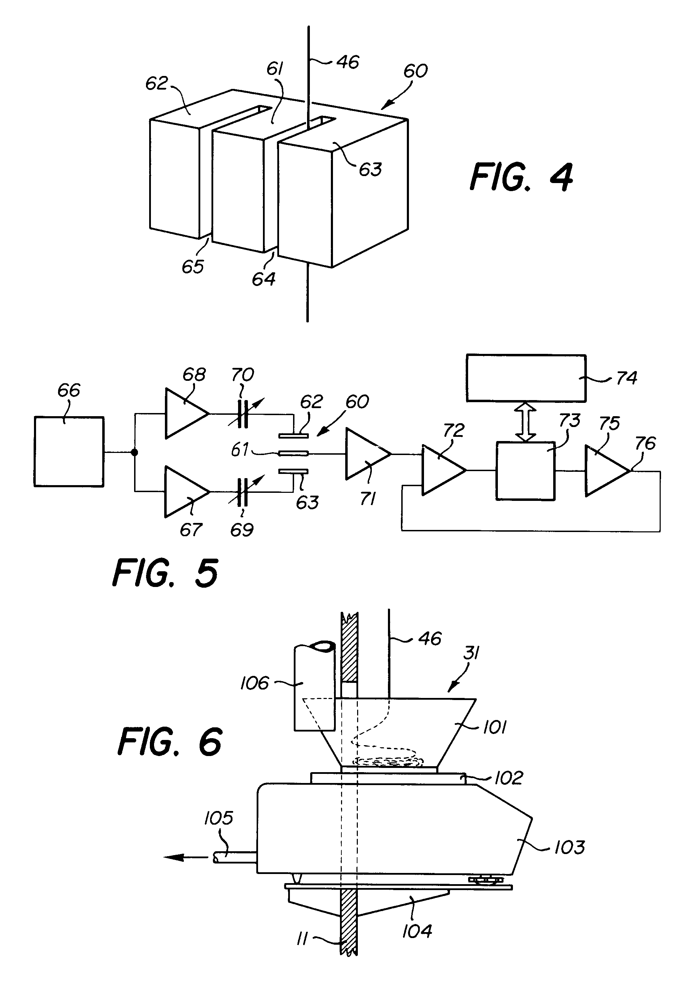

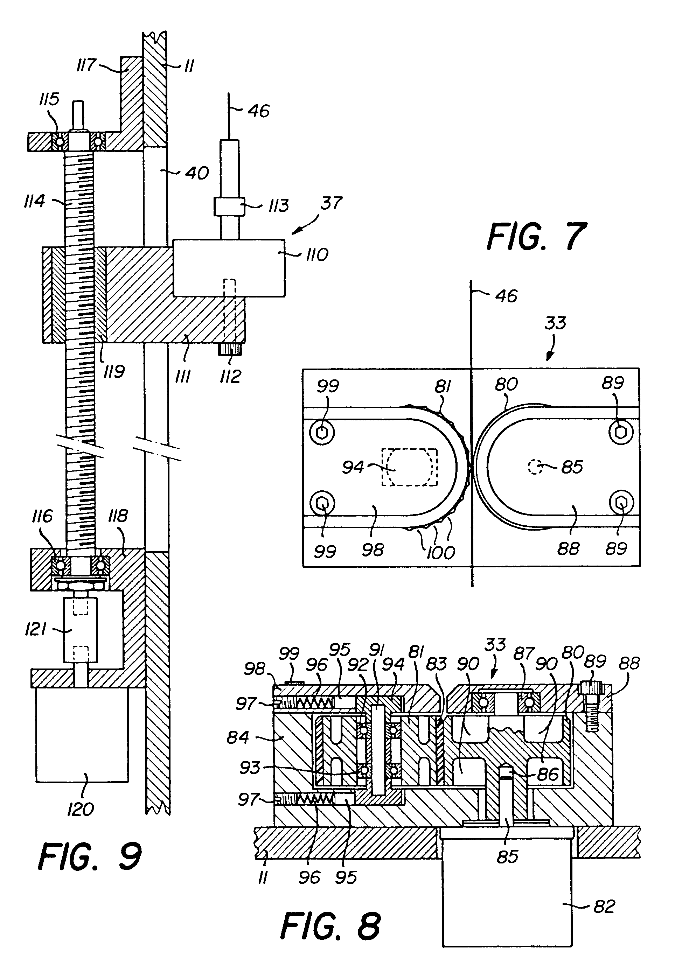

Le premier module de mesure monté verticalement à gauche de l'appareil représenté par la fig. 1, comporte un dispositif pour la détermination du titre ou masse linéique, ce dispositif comprenant une balance de précision 31 décrite plus en détail en référence à la fig. 6, un dispositif pour la détermination de la régularité de la masse linéique, comportant un condensateur double 32 et son circuit associé décrit plus en détail en référence aux figures 4 et 5, ainsi qu'un mécanisme d'entrainement 33 décrit plus en détail en référence aux figures 6 et 7.

Le second module de mesure, monté verticalement à droite dans l'appareil représenté par la fig. 1, comporte un dispositif 34 comprenant un moteur d'entrainement pas-à-pas 35 destiné à entrainer en rotation une pince 36 agencée pour pincer une extrémité d'une éprouvette de fil dont on souhaite mesurer la torsion et un dispositif 37 également pourvu d'une pince 38 pour maintenir l'autre extrémité de l'éprouvette de fil dont on souhaite mesurer la torsion. Le dispositif 37 est en fait mobile axialement entre sa position représentée en trait plein et une position 39, représentée en trait interrompu. Le déplacement de ce dispositif est possible grâce à une fente longitudinale 40 ménagée dans la plaque de montage 11. Ce dispositif sera décrit plus en détail en référence à la fig. 9.

Comme mentionné précédemment, les fils proviennent d'un support, appelé râtelier et représenté par la fig. 2, se composant d'un châssis 41 réalisé par exemple à l'aide de, profilés métalliques soudés. Le châssis 41 porte, dans le présent cas, quatre traverses 42 portant chacune une série de tiges 43 sur lesquelles on enfile respectivement une bobine 44 de fil que l'on veut tester. Comme le montre la figure, les bobines peuvent avoir des formes et des dimensions très variées et leur nombre peut varier considérablement en fonction des dimensions du râtelier. En pratique, il est avantageux de prévoir la possibilité de stocker jusqu'à vingt-quatre bobines de fil. Au-dessus de chaque bobine est disposé un tube d'aspiration 45 qui communique avec un des tubes flexibles 16 qui aboutissent à l'appareil de contrôle. L'air comprimé, destiné à aspirer un fil 46 amené manuellement à l'entrée du tube d'aspiration 45 correspondant, et à l'accompagner jusque dans le caisson 10, est injecté latéralement dans les tubes 16, comme le montrent les flèches E. La commande de l'aspiration se fait au moyen de manettes 47 montées sur la poutrelle transversale supérieure du châssis 41. Ces manettes commandent des vannes qui mettent les conduits 48, qui injectent tangentiellement de l'air comprimé dans les tubes 16, en communication avec une source d'air sous pression (non représentée).

Bien que non indispensable au fonctionnement de l'appareil de contrôle, le râtelier constitue un accessoire fort utile pour tester automatiquement un nombre important de fils différents.

Lorsque les fils sont injectés au haut de l'appareil 10, un opérateur les met en place entre les mâchoires 49 et 50 d'une pince 51, illustrée par la fig. 3. Chaque pince 51 est constituée par deux mâchoires qui se composent chacune d'une partie arrière cylindrique 52 engagée dans un alésage 53 d'un support 54, et d'une partie antérieure 55 profilée en forme d'obus, les deux parties étant séparées par une zone 56 de diamètre réduit. Les deux mâchoires 49 et 50 qui forment la pièce 51 sont disposées côte à côte, et respectivement engagées, avec un peu de jeu, dans deux alésages 53 communiquant entre eux. Un aimant permanent 57, logé dans un évidement approprié ménagé dans le support 54, réalisé en un matériau amagnétique tel que l'aluminium ou une matière synthétique, assure l'auto-alignement des deux pièces 49 et 50 et leur serrage. Un fil 46 amené au travers d'un conduit 16 fixé dans une entaille semi-cylindrique 58 du support 54, est pincé entre les mâchoires 49 et 50 de la pince 51, grâce à l'effet auto-alignant exercé par l'aimant 57 et au jeu existant entre les extrémités cylindriques 52 de ces mâchoires et les alésages 53 qui les logent. Chaque support 54 comporte quatre pinces 51. L'ensemble des supports 54 est disposé sur l'arc de cercle 30 de l'appareil illustré par la fig. 1.

Lorsque les fils sont pincés entre les mâchoires des pinces 51, ils se trouvent en position d'attente pour être pris en charge par le manipulateur 17 et être introduits dans les différents instruments de mesure.

L'un de ces instruments de mesure est représenté par la fig. 4. Il S'agit d'un condensateur double associé à un circuit de mesure. représenté par la fig. 5 qui permettent de comparer les masses de longueurs déterminées (par exemple 5mm) de fil, par une mesure capacitive. A cet effet, le condensateur double 60 comporte une plaque centrale 61 et deux plaques latérales 62 et 63 qui définissent deux couloirs 64 et 65. Le couloir 64 ne contient que de l'air alors que le couloir 65 est traversé par le fil 46.

Comme le montre la fig. 5, ce condensateur 80 est monté dans un pont capacitif dont l'état d'équilibre dépend de la masse du fil dans le couloir 64. Le circuit se compose d'un oscillateur 66 raccordé à deux amplificateurs 67 et 68 agencés pour fournir aux deux plaques latérales 62 et 63, des signaux en opposition de phase. Deux condensateurs de réglage 69 et 70 permettent d'ajuster la capacité à vide du condensateur double 60. Lorsque les deux capacités du condensateur double 60 sont égales, aucune tension n'apparait à la plaque centrale 61. Par contre, si les deux capacités sont différentes, la tension de la plaque centrale est amplifiée par un amplificateur 71, transmise à l'une des entrées d'un convertisseur analogique/numérique 72, démodulée par un démodulateur 73 et transmise à l'unité centrale de traitement des données, constituée par un micro-ordinateur 74. De cette manière, les mesures de régularité de masse linéique sont effectuées sur une grande longueur, par exemple 100 m, et suivies d'une mémorisation et d'un traitement statistique. Un circuit de contre-réaction qui permet de masquer les déséquilibres du pont, comporte un convertisseur numérique-analogique 75 dont la sortie 76 est connectée à l'autre entrée du convertisseur analogique-numérique 72.

Pour effectuer ces mesures, le fil 46 doit défiler à relativement grande vitesse dans la fente 64. Ce défilement contrôlé est obtenu grâce au dispositif 33 illustré en détail par les figures 7 et 8.

Ce dispositif comporte essentiellement un rouleau d'entrainement 80 et un rouleau presseur 81. Le rouleau d'entrainement 80 est entrainé par un moteur pas-à-pas 82 dont on connait avec précision la vitesse, et a un diamètre qualibré, de telle manière que l'on puisse déduire avec toute la précision souhaitée, la longueur du fil entrainé par ce dispositif. Le rouleau presseur comporte un revêtement anti-dérapant 83 qui prend appui sur la surface périphérique du rouleau d'entrainement 80. Le dispositif comporte un berceau 84 en forme de U qui est porté par la plaque de montage 11. L'arbre d'entrainement 85 du moteur 82 est monté dans un alésage axial 86 du rouleau 80. L'axe de ce dernier est, par ailleurs, porté par un palier 87 monté à l'intérieur d'un évidement ménagé dans une plaque de recouvrement 88 rendue solidaire du berceau 84 par des vis 89. Le rouleau 80 comporte avantageusement des évidements intérieurs 90 destinés à diminer son inertie. Le rouleau 81 comporte un axe 91 porté par deux paliers 92 et 93. Ces deux paliers sont montés dans un bloc coulissant 94 qui est soumis par l'intermédiaire de deux plots 95 à l'action de deux ressorts 96 dont la tension peut être réglée par deux vis de réglage 97. Une plage de recouvrement 98 rendue solidaire du berceau 84 par des vis 99 assure la même fonction de protection que la plaque 88. Le bord périphérique extérieur du rouleau presseur 81 présente un certain nombre d'entailles 100, dont le rôle est de faciliter d'une manière connue en soi, l'introduction automatique d'un fil 46 entre les deux rouleaux 80 et 81.

Après son passage à travers les rouleaux 80 et 81, le fil 46 se dépose dans un réceptacle 101 placé sur le plateau 102 d'une balance de précision 103 illustrée par la fig. 6. Cette balance est portée par une console 104 solidaire de la plaque de montage 11. Cette balance permet de déterminer le titre, c'est-à-dire la masse linéique du fil en pesant des longueurs contrôlées, déposées dans le réceptacle 101. Une liaison 105 assure la transmission des résultats des pesées successives à l'unité centrale de traitement des données et/ou à la mémoire, en vue du traitement statistique de ces résultats et/ou de leur archivage.

L'évacuation des éprouvettes testées se fait par aspiration au moyen d'une hotte ou d'un tube d'aspiration 106.

Les deux mesures de titre et de régularité du titre s'effectuent sur le même module. Le dispositif d'entrainement est commun aux deux organes de mesure proprement dits. Le fil est disposé verticalement au cours de ces mesures et est évacué automatiquement après les tests.

Le second module se compose, comme mentionné précédemment, d'un organe permettant de déterminer la torsion des éprouvettes de fil et leur propriétés dynamométriques. La détermination de la torsion de filés fait notamment l'objet d'une norme "AFNOR" et d'une thèse de doctorat, soutenue le 12 jui 1981 par Jean-Yves DREAN devant la Commision d'Examen de l'Université de Haute Alsace, et ne sera pas décrite plus en détail dans ce texte. Cette détermination implique une préépreuve, et en particulier la connaissance du titre qui sert à la détermination de la prétension. Du fait de la commande et de la gestion informatisées, la détermination du titre peut être entièrement automatisée. Cette détermination s'effectue grâce au dispositif 34 comprenant le moteur pas-à-pas 35 qui entraine dans un sens ou dans l'autre, la pince 36 qui tient l'éprouvette à tester.

La fig.9 représente une vue en coupe partielle de l'appareil de contrôle illustrant essentiellement le dispositif 37. Ce dispositif se compose d'un capteur de force 110 fixé sur un support 111, par exemple à l'aide d'une vis 112, et portant une pince 113 qui sert à fixer l'extrémité inférieure d'une éprouvette de fil dont on veut mesurer la torsion et/ou les propriétés dynamométriques. Le support 111 est mobile en translation verticale le long d'une vis 114 rotative sur deux paliers 115 et 116 respectivement portés par deux supports : une équerre 117 et une pièce 118 en forme de U solidaires de la plaque de montage 11. A cet effet, il porte un manchon fileté 119 qui se déplace en translation en entrainant le support 111 lorsque la vis tourne sur ses paliers-supports, entrainée par un moteur pas-à-pas 120 par l'intermédiaire d'un cardan 121.

Ce dispositif est utilisé à la fois pour effectuer les mesures de torsion et pour effectuer les mesures des propriétés dynamométriques. Dans le premier cas, il est utilisé en combinaison avec le moteur 34 qui fait tourner la pince 36 pour torsader successivement le fil dans un sens et dans l'autre, la pince 113 ayant une position en hauteur fixe, et dans le second cas, la pince 36 reste fixe et la pince 113 est abaissée jusqu'à la rupture du fil 46. Comme le montre la figure, seule la pince portée par le capteur de force 110 apparâit à l'avant de la plaque de montage 11. Le support 111 coulisse verticalement à travers la fente 40. Les organes mécaniques responsables du déplacement de la pince 113 sont disposés à l'arrière de la plaque de montage 11.

On constate que les moyens utilisés reposent sur la mise en oeuvre d'un système totalement informatisé, aussi bien pour la gestion des systèmes mécaniques à mettre en oeuvre pour une automatisation complète, que pour l'interprétation de résultats préliminaires, éventuellement directement, afin de permettre le choix de la variante de méthode à appliquer, ainsi que pour l'interprétation statistique des résultats au fur et à mesure de leur obtention pour diriger l'avancement ou l'arrêt des séquence de déterminations selon des instructions données initialement.

De plus, la numérisation de toutes les données permet l'obtention de paramètres calculés par ordinateur qui échappent généralement aux contrôles classiques, comme par exemple en dynamométrie : les modules, la capacité de travail... qui sont des paramètres beaucoup plus significatifs (et donc plus intéressants en pratique) pour la prévision des comportements à l'usage que les simples résistances et allongements à la rupture donnés classiquement.

Par ailleurs, le traitement informatique des données permet l'exploitation statistique poussée non seulement pour donner la moyenne, l'écart-type et/ou le coefficient de variation, mais aussi d'autres pa-ramètres statistiques comme par exemple les coefficients de symétrie β₁ et d'étalement β₂ de Pearson, indicateurs du type de distribution à laquelle on a affaire.

La combinaison, selon une séquence appropriée des différents contrôles, permet au système de fournir les données nécessaires à la poursuite de certains d'entre eux : ainsi la déterminationnn automatique en premier lieu du titre, fournit la base pour le calcul et l'application automatique de la tension préalable.

Enfin l'informatisation complète de l'ensemble de mesures permet l'initialisation selon le mode conversationnel pour fixer initialement à la mise en route le ou les types de contrôle désirés, le nombre de mesures et/ou la précision statistique désirée, le nombre de bobines ou la combinaison : nombre d'essais par bobines, nombre de bobines, ainsi que tout autre ordre ou contrainte voulu ou nécessaire.

Cette information permet évidemment la sortie des informations collationnées et traitées sur n'importe quel système intermédiaire : imprimante, stockage sur mémoire etc...

La présente invention n'est, bien entendu, pas limitée aux formes de réalisation décrites à titre d'exemples non limitatifs, mais peut subir de nombreuses modifications et se présenter sous diverses variantes évidentes pour l'homme de l'art. En particulier, l'appareil de l'invention n'est pas limité à deux modules de mesure, mais peut être complété, par exemple, par un troisième module qui détermine la structure superficielle des fils, leur pilosité, leur voluminosité, etc...

## Revendications

1. Appareil de contrôle automatique d'un fil textile, caractérisé en ce qu'il comporte un caisson (10) contenant d'une part deux modules de mesure pourvus, l'un, de moyens (31, 32) pour effectuer des mesures sur ce fil permettant de déterminer son titre et sa régularité de masse linéique, l'autre, de moyens (35, 38, 39) pour effectuer des mesures sur ce fil permettant de déterminer sa torsion et ses propriétés dynamométriques, et d'autre part un manipulateur et une pince pour prélever des éprouvettes de fil d'une position d'attente et pour les amener vers ledit premier ou ledit deuxième module, et en ce qu'il comporte en outre des moyens utilisant le résultat de la détermination du titre pour calculer et appliquer automatiquement une tension préalable au fil lors des mesures permettant la détermination de la torsion et des propriétés dynamométriques, et des moyens pour réitérer automatiquement ces mesures sur une série d'éprouvettes d'un même fil, ainsi que des moyens pour enregistrer en mémoire les résultats de ces mesures et des moyens informatiques pour traiter ces résultats en vue de leur exploitation statistique et de leur archivage.

2. Appareil selon la revendication 1, caractérisé en ce que le premier module comporte un organe de mesure (32), par voie capacitive, de la régularité du titre du fil, un organe de mesure (32) du titre de ce fil, et un dispositif d'entraînement (33), ces trois composants étant disposés sur une même verticale et agencés pour coopérer à la détermination simultanée du titre d'un fil et de la régularité de ce même titre.

3. Appareil selon la revendication 2, caractérisé en ce que l'organe de mesure (32) de la régularité du titre comporte un condensateur double (60) comprenant une plaque centrale (61) et deux plaques latérales (62) et (63), définissant deux couloirs (64) et (65) dont l'un (65) est traversé par un fil (46) à tester, ainsi qu'un circuit électronique de commande comportant un oscillateur (66) agencé pour appliquer sur les plaques latérales (62, 63) une tension alternative en opposition de phase, un amplificateur (71) et un démodulateur (73) pour détecter les variations de tension sur la plaque centrale (61) et un circuit de contre-réaction comprenant un convertisseur analogique-numérique (72) et un convertisseur numérique-analogique (75) pour masquer les déséquilibres du pont capacitif comprenant les plaques du condensateur double (60), le démodulateur (73) étant connecté à une unité centrale de traitement des données (74), en vue du traitement statistique et de l'archivage des résultats des mesures.

4. Appareil selon la revendication 1, caractérisé en ce que le dispositif d'entrainement (33) comporte un berceau (84) fixé à la plaque de montage (11), un rouleau d'entrainement (80) couplé à un moteur d'entrainement pas-à-pas (82) et un rouleau presseur (81) pourvu d'un revêtement antidérapant (83), ce rouleau presseur étant pourvu de moyens (94, 96, 97) pour assurer une pression constante de sa surface périphérique contre la surface périphérique du rouleau d'entrainement (80).

5. Appareil selon la revendication 4, caractérisé en ce que le rouleau presseur comporte le long de son rebord périphérique extérieur au moins une entaille (100) pour faciliter l'insertion automatique d'un fil (46) entre les deux rouleaux (80, 81).

6. Appareil selon revendication 1, caractérisé en ce que l'organe de mesure (31) du titre comporte une balance de précision (103) posée sur une console (104) solidaire de la plaque de montage (11), équipée d'un réceptacle (101) pour recevoir des longueurs contrôlées de fil (46), et d'une sortie (105) des signaux correspondant aux mesures successives, ces signaux étant transmis a l'unité centrale de traitement des données en vue de l'exploitation statistique et de l'archivage des résultats des mesures.

7. Appareil selon revendication 1, caractérise en ce que le second module est conçu pour déterminer la torsion et les propriétés dynamométriques d'une éprouvette de fil et comporte un dispositif (34) comprenant un moteur pas-à-pas (35) portant une pince (36) entrainée en rotation dans un sens ou dans l'autre par ce moteur, et conçu pour retenir une extrémité d'une éprouvette de fil à tester, un dispositif (37) comprenant une pince (38) et un capteur de force (110), et des moyens mécaniques pour déplacer le dispositif (37) en hauteur le long d'une fente verticale (40) ménagée dans la plaque de montage (11).

8. Appareil selon revendication 7, caractérise en ce que les moyens pour déplacer le dispositif (37) en hauteur comportent un moteur pas-à-pas (120), et une vis verticale (114), entraînée en rotation par ce moteur et couplée au dispositif (37) pour assurer son déplacement vertical.

9. Appareil selon revendication 1, caractérisé en ce qu'il comporte des moyens (106) pour aspirer automatiquement les éprouvettes de fil après les mesures.

10. Appareil selon revendication 1, caractérisé en ce qu'il comporte un déflecteur (9) conçu pour permettre de définir deux zones séparées, dont l'une contient une éprouvette de fil testée par le premier module et dont l'autre contient une éprouvette de fil testée par le second module.

11. Appareil selon revendication 1, caractérisé en ce que le manipulateur (17) est mobile selon trois axes orthogonaux, et comprend un disque rotatif (18), un bloc de guidage (22) monté sur deux rails (23, 24) fixés perpendiculairement à ce disque de telle manière que ce bloc puisse coulisser le long de ces rails, une tige (21) coulissant à l'intérieur du bloc de guidage (22), un bras de manipulation (20) monté à l'extrémité libre de la tige (21) et portant la pince (29) pour prélever des fils de leurs positions d'attente et pour les amener vers un organe de mesure ou pour couper une longueur prédéterminée de ces fils.

12. Appareil selon revendication 1, caractérisé en ce qu'il comporte un râtelier formé d'un châssis (41) portant un ensemble de tiges (43) agencées pour recevoir des bobines de fil (44) à tester, des tubes d'aspiration (45) pour aspirer ces fils et pour les amener au travers de tubes flexibles (16) à l'intérieur de l'appareil de contrôle.

13. Procédé de contrôle automatique d'un fil textile, dans lequel on effectue automatiquement un ensemble de mesures sur ce fil au moyen d'un même appareil mettant en oeuvre l'appareil selon l'une quelconque des revendications 1 à 12, caractérisé en ce que ces mesures permettent de déterminer le titre de ce fil, sa régularité de masse linéique, sa torsion et ses propriétés dynamométriques, en ce que l'on utilise le résultat de la détermination du titre pour calculer et appliquer automatiquement une tension préalable au fil lors des mesures permettant la détermination de la torsion et des propriétés dynamométriques, en ce que l'on réitère automatiquement les mesures et qu'on enregistre en mémoire les résultats de ces mesures pour les traiter par voie informatique en vue de leur exploitation statistique et de leur archivage.

14. Procédé de contrôle selon la revendication 13, caractérise en ce que l'on effectue les mesures pour déterminer le titre et la régularité de masse linéique au moyen d'une première éprouvette dudit fil et en ce que l'on effectue les mesures pour déterminer la torsion et les caractéristiques dynamométriques au moyen d'une deuxième éprouvette de ce fil.

15. Procédé selon la revendication 13, caractérisé en ce que l'on introduit une série d'éprouvettes de fil dans l'appareil de telle manière que leurs extrémités soient localisées chacune dans une position d'attente d'où elles peuvent être reprises pour être introduites successivement dans un des modules de mesure, en ce que l'on effectue successivement pour chaque fil une série de mesures répétées au moyen des organes de mesures d'un même module, et en ce que l'on arrête cette série de mesures soit lorsqu'un nombre prédéterminé de mesures a été effectué, soit lorsque la précision statistique est atteinte pour un nombre de mesures inférieur audit nombre prédéterminé.

16. Procédé selon la revendication 15, caractérisé en ce que l'on introduit automatiquement les éprouvettes de fil dans l'appareil à l'aide de jets d'air et en ce que l'on fixe manuellement les extrémités libres de ces fils dans leurs positions d'attente respectives.

17. Procédé selon la revendication 13, caractérisé en ce que l'on dispose les éprouvettes verticalement au cours des mesures effectuées dans chacun des modules.

18. Procédé selon la revendication 13, caractérisé en ce que l'on évacue automatiquement, par aspiration, les déchets ainsi que les éprouvettes ayant fait l'objet des mesures.

## Claims

1. A device for the automatic testing of textile thread, characterized in that it comprises a box (10) containing on the one hand, two measuring modules, one of which is fitted with the means (31, 32) for measuring this thread in order to determine its count and the eveness of its linear mass, and on the other hand, the means (35, 38, 39) for measuring this thread in order to determine its twist and its dynamometric properties, and also a manipulator and forceps for removing samples of thread from a waiting position and taking them to the said first or second module, and which also comprises the means for using the result of the count determination in order to calculate and automatically apply a preset tension to the thread during measurements to determine the twist and dynamometric properties, and the means for retaking these measurements automatically from a series of samples of the same thread, as well as the means for recording and saving the results of these measurements, and a data processing means for processing these results with a view to their statistical exploitation and memory storage.

2. A device according to claim 1, characterized in that the first module includes a device for measuring (32) the eveness of the thread by capacitive means, a device for measuring (32) the count of this thread, and a drive mechanism (33), these three components being disposed along the same vertical and adapted to work in cooperation for the simultaneous determination of the count of a thread and the eveness of this same count.

3. A device according to claim 2, characterized in that the device for measuring (32) the eveness of the count includes a double condenser (60) having a central plate (61) and two lateral plates (62) and (63), defining two passageways (64) and (65), one of which (65) is crossed by a thread (46) to be tested, and also an electronic control circuit including an oscillator (66), set to apply alternating voltage in opposition of phase, an amplifier (71) and a demodulator (73) to detect voltage variations on the central plate (61) and a negative feed-back circuit comprising an analog-digital convertor (72) and a digital-analog convertor (75) to accomodate the desiquilibrium of the connecting-circuit comporting the plates of the double condenser (60), the demodulator (73) being connected to a central processing unit (74) for statistical processing and memory storage of the measurement results.

4. A device according to claim 1, characterized in that the drive means (33) includes a U-shape receptacle (84) fixed to the mounting plate (11), a drive cylinder (80) coupled to a stepper drive motor (82) and a pressure cylinder (81) fitted with an anti-slip cover (83), this pressure cylinder being fitted with the means (94, 96, 97) for ensuring constant pressure of its surface periphery with the surface periphery of the drive cylinder (80).

5. A device according to claim 4, characterized in that the pressure cylinder has at least one opening (100) along the length of its external periphery, to facilitate the automatic insertion of a thread (46) between the two cylinders (80, 81).

6. A device according to claim 1, characterized in that the device (31) for measuring count includes a precision balance (103) set on a flat extension (104) which forms part of the mounting plate (11), equipped with a receptacle (101) to receive controlled lengths of thread (46), and a signal output (105) corresponding to the successive mesaurements, these signals being transmitted to the central processing unit for statistical processing and memory storage of the measurement results.

7. A device according to claim 1, characterized in that the second module is designed to determine the twist and dynamometric properties of a sample of thread and includes a device (34) composed of a stepper driver (35) with forceps (36) carried in rotation in one direction or the other by this motor, and designed to hold the extremity of a sample of the thread to be tested, a device (37), composed of forceps (38) and a tension sensor (110), and the mechanical means of moving the device (37) vertically along a vertical slit (40) in the mounting plate (11).

8. A device according to claim 7, characterized in that the means for moving the device (37) vertically comprises a stepper motor (120), and a vertical screw (114), drawn in rotation by this motor and coupled to the device (37) in order to ensure its vertical displacement.

9. A device according to claim 1, characterized in that it includes the means for automatically sucking up (106) the thread samples after measurement.

10. A device according to claim 1, characterized in that it comprises a deflector (9) designed to allow the definition of two separate zones, one of which contains a thread sample tested by the first module, and the other of which contains a thread sample tested by the second module.

11. A device according to claim 1, characterized in that the manipulator (17) is mobile along three orthogonal axes, and includes a rotating disc (18), a guide block (22) mounted on two rails (23, 24) fixed perpendicularly to this disc in such a way that the disc can slide along these rails, a stem (21) sliding inside the guide block (22), a manipulation arm (20) mounted on the free end of the stem (21) and carrying the forceps (29) for removing samples from their waiting positions and taking them to a measuring device or for cutting a predetermined length of this thread.

12. A device according to claim 1, characterized in that it comprises a rack formed by a body (41) carrying a set of spindles (43) adapted to receive bobbins of thread (44) for testing, suction tubes (45) to suck up these threads and take them through flexible tubes (16) to the inside of the testing device.

13. A process for the automatic testing of a textile thread, in which a set of measurements is automatically taken from this thread, using the same device, setting the device into operation according to any of claims 1 to 12, characterized in that these measurements allow the determination of the count of this thread, the eveness of its linear mass, its twist and its dynamometric properties, and in that the result of the determination of count is used to calculate and automatically apply a preset tension to the thread during measurements for the determination of twist and dynamometric properties, and in that the measurements are repeated automatically and that the results are recorded in memory for data processing with a view to statistical exploitation and memory storage.

14. A process according to claim 13, characterized in that the measurements taken for the determination of count and the eveness of the linear mass use a first sample of the said thread and in that the measurements taken for the determination of twist and the dynamometric characteristics of the said thread use a second sample of this thread.

15. A process according to claim 13, characterized in that a series of thread samples is introduced into the device in such a manner that each of their extremities is localized in a waiting position from where they can be removed for successive introduction into one of the measuring modules, and in that a series of measurements is repeated for each successive thread, using the measuring device of the same module, and in that this series of measurements is stopped either when a predetermined number of measurements has been taken, or when statistical precision is reached for a lower number of measurements than the said predetermined number.

16. A process according to claim 13, characterized in that the thread samples are automatically introduced into the device by means of air jets and in that the free extremities of these threads are fitted manually into their respective waiting positions.

17. A process according to claim 13, characterized in that the samples are positioned vertically during the measurements carried out in each of the modules.

18. A process according to claim 13, characterized in that the waste and the samples which have been tested are evacuated automatically by suction.

## Patentansprüche

1. Automatisches Kontrollgerät für ein Textilgarn, dadurch gekennzeichnet, dass es einen Kasten (10) enthält; dieser besitzt einerseits zwei Messmodulen, einer mit Mitteln (31,32) versehen, die es ermöglichen, Messungen auf diesem Garn zu unternehmen und seinen Titer sowie die Gleichmässigkeit seiner längenbezogenen Masse festzustellen; der andere, mit Mitteln (35, 38, 39) versehen, die es ermöglichen, Messungen auf diesem Garn zu unternehmen um seine Drehung und dynamometrischen Eigenschaften festzulegen; andererseits beinhaltet dieser Kasten ebenfalls einen Manipulator und eine Zange, die es ermöglichen Garnprobegläser aus einer Bereitschaftsstellung zu entnehmen und sie zum besagten ersten Modul oder besagten zweiten Modul zuzuführen und dass es ausserdem Mittel besitzt, die das Titerbestimmungsresultat verwenden, um automatisch eine vorherige Spannung des Garns anlässlich der Messungen zu berechnen und anzuwenden, wobei die Drehungs-und-dynamometrischen Eigenschafts-Ermittlungen durchgeführt werden können; gleichzitig besitzt es Mittel, um automatisch diese Messungen auf einer Probegläserserie desselben Garns zu widerholen sowie Mittel um die Resultate dieser Messungen zu speichern, elektronische Datenverarbeitungsmittel um diese Resultate in der Absicht, sie statistich zu verwerten und sie zu archivieren, zu verarbeiten.

2. Gerät nach Anspruch 1, dadurch gekennzeichnet, dass der erste Modul ein kapazitätmässiges Messorgan (32) für die Gleichmässigkeit des Garntiters sowie ein Messorgan (32) für den Garntiter und eine Antriebsvorrichtung (33) beinhaltet, wobei diese drei Komponenten sich auf derselben Senkrechte befinden und so ausgerüstet sind, dass eine simultane Garntiter-und-Titergleichmässigkeitsbestimmung entsteht.

3. Gerät nach Anspruch 2, dadurch gekennzeichnet, dass das Messorgan (32) der Titergleichmässigkeit einen Doppelabscheider (60) mit einer Zentralplatte (61) und zwei seitlichen Platten (62, 63) beinhaltet; diese Platten bezeichnen zwei Trichter (64, 65), wovon einer (65) von einem Prüfgarn (46) durchgedrungen wird; das Messorgan besitzt ebenfalls eine Steuerelektronik mit einem Oszillator (66), der dazu ausgestattet ist, um eine Wechselspannung in entgegengesetzter Phase auf die seitlichen Platten (62,63) aufzudrücken; das Messorgan besitzt ausserdem einen Verstärker (71) und einen Demodulator (73) um die Spannungsänderungen auf der Zentralplatte (61) zu entdecken sowie einen Gegenkopplungsstromkreis, der einen Analog-Digital-Umsetzer (72) und einen Digital-Analog-Umsetzer (79) enthält um die Fehlanpassungen der Kapazitätsmessbrücke, die die Doppelabscheider-Platten (60) einschliesst zu verdecken; dabei wird der Demodulator (73) zum Zweck von der statistischen Verarbeitung und der Messresultaten-Archivierung an eine Datenverarbeitungs-Zentraleinheit (74) gekuppelt.

4. Gerät nach Anspruch 1, dadurch gekennzeichnet, dass die Antriebsvorrichtung (33) eine Wiege (84) enthält, die an der Montageplatte (11) befestigt ist, sowie eine Zugwalze (80), die an einen Antriebschrittmotor (82) gekoppelt ist und eine Druckwalze (81) mit einem rutschfest Überzug und eine Druckwalze die Mittel (94, 96, 97) besitzt, um einen konstanten Druck ihrer peripherischen Oberfläche gegen die Umfangsoberfläche der Zugwalze (80) zu versichern.

5. Gerät nach Anspruch 4, dadurch gekennzeichnet, dass die Druckwalze längs ihrem peripherischem Aussenrand mindestens einen Einschnitt (100), der den automatischen Garnschlag (46) zwischen beiden Walzen (80, 81) erleichtert, besitzt.

6. Gerät nach Anspruch 1, dadurch gekennzeichnet, dass das Titermessorgan (31) eine Präzisionswaage (103) beinhaltet, die sich auf einer zur Montageplatte (11) gehörenden Konsole (14) befindet; diese Präzisionswaage besitzt einen Auffang (101) für den Empfang der geprüften Garnlängen (46) und einen Ausgang (105) dessen Signale den aufeinanderfolgenden Messungen entsprechen - wobei diese Signale an die Datenverarbeitungs-Zentraleinheit zum Zweck von der statistichen Verarbeitung und der Archivierung der Messresultate ûbergeben werden.

7. Gerät nach Anspruch 1, dadurch gekennzeichnet, dass der zweite Modul dazu dient, die Drehung und die dynamometrischen Eigenschaften eines Garnprobeglases festzulegen; dieser Modul besitzt eine Vorrichtung (34) mit einem Schrittmotor (35), dessen Zange (36) rotierend in eine Richtung oder in die andere durch diesen Motor in Bewegung gebracht wird; ausserdem dient dieser Modul dazu, ein Probeglasende eines zu prüfenden Garns und eine mit einer Zange (38) und einem Kraftmessgerät (110) versehenen Vorrichtung (37) festzuhalten, und besitzt mechanische Mittel um diese vorrichtung (37) oberhalb, längs einer in einer Montageplatte (11) vorgesehenen senkrechten Ritze (40) in Bewegung zu setzen.

8. Gerät nach Anspruch 7, dadurch gekennzeichnet, dass die Mittel zur oberhalben Fortbewegung der Vorrichtung (37) einen Schrittmotor (120) und eine durch diesen Motor rotierend angetriebene senkrechte Schraube (114) besitzen; um ihre senkrechte Verschiebung zu versichern, ist diese Schraube an die Vorrichtung (37) gekoppelt.

9. Gerät nach Anspruch 1, dadurch gekennzeichnet, dass es Mittel (106) beinhaltet, die es ermöglichen, automatisch die Garnprobegläser nach der Messungen abzusaugen.

10. Gerät nach Anspruch 1, dadurch gekennzeichnet, dass es einen Abweiser (9) enthält, der dazu dient zwei getrennte Zonen zu definieren, wobei eine Zone ein Probeglas mit durch den ersten Modul geprüftem Garn beinhaltet und die andere Zone ein Probeglas mit durch den zweiten Modul geprüftem Garn enthält.

11. Gerät nach Anspruch 1, dadurch gekennzeichnet, dass der Manipulator (17) mittels drei orthogonalen Achsen beweglich ist und eine Drehungsscheibe (18) sowie einen auf zwei Schienen (23, 24) montierten Lenkungsblock (22) enthält; die besagte Schienen sind senkrecht zu dieser Schreibe so fixiert, dass dieser Block längs dieser Schienen gleiten kann, der besagte Manipulator besitzt ebenfalls einen innerhalb des Lenkungsblocks (22) gleitenden Stift (21) und einen auf dem freien Stiftende (21) montierten Handhabungsarm (20), der zur Entnahme der Garne aus ihrer Bereitschaftsstellung und zu deren Zuführung zu einem Messorgan, oder zum Abschneiden dieser Garne auf eine vorbestimmte Länge, mit einer Zange (29) ausgerüstet ist.

12. Gerät nach Anspruch 1, dadurch gekennzeichnet, dass es ein Rahmengatter (41) mit einem zum Empfang von Prüfgarnspulent (44) ausgerüstetem Stiftesatz (43) sowie Absaugerohre (45) um diese Garne abzusaugen und sie querdurch biegsame Rohre (16) in das Kontrollgerät zuzuführen, beinhaltet.

13. Verfahren für die automatische Kontrolle eines Textilgarns in dem man automatisch eine Messungengesamheit auf diesem Garn mittels eines einzigen Geräts nach irgendeinem Anspruch von 1 bis 12 durchführt, dadurch gekennzeichnet, dass diese Messungen die Ermittlung dieses Garntiters sowie die Gleichmässigkeit der längenbezogenen Masse, seine Drehung und seine dynamometrische, Eigenschaften ermöglichen; dazu muss man das Titerbestimmungsresultat verwenden um automatisch eine vorherige Spannung des Garns anlässlich der Drehungs-und-dynamometrischen Eigenschaftsermittlungen zu berechnen und anzuwenden, indem man diese Messungen automatisch wiederholt und die Resultate dieser Messungen speichert um sie zum Zweck ihrer statistischen Verarbeitung und ihrer Archivierung mittels elektronischer Datenverarbeitung zu bearbeiten.

14. Kontrollverfahren nach Anspruch 13, dadurch gekennzeichnet, dass man Messungen durchführt, um den Titer und die Gleichmässigkeit der längenbezogenen Masse mittels eines ersten Probeglases des besagten Garns festzulegen; und, dass man Messungen zur Feststellung der Drehung und der dynamometrischen Eigenschaften mittels eines zweiten Probeglases dieses Garns unternimmt.

15. Verfahren nach Anspruch 13, dadurch gekennzeichnet, dass man eine Garnprobeglasserie so in das Gerät einführt, dass ihre Enden jeweils in einer Bereitschaftsstellung lokalisiert werden können; man kann sie dann wieder herausholen, um sie nacheinander in einen der Messmodulen einzuführen, wobei man nacheinander für jedes Garn eine Serie von wiederholten Messungen mit den Messorganen eines und desselben Moduls unternimmt und wobei man diese Mess-Serie entweder nach Durchführung einer vorbestimmten Anzahl von Messungen, oder wenn die statistische Präzision bei einer im Vergleich zur vorbestimmten Anzahl minderen Anzahl von Messungen erreicht wurde, einstellt.

16. Verfahren nach Anspruch 15, dadurch gekennzeichnet, dass man die Garnprobegläser mit Hilfe von Luftstrahlen in das Gerät automatisch einführt und indem man die freien Garnenden in ihrer jeweiligen Bereitschaftsstellung manuell fixiert.

17. Verfahren nach Anspruch 13, dadurch gekennzeichnet, dass man die Probegläser anlässlich der durchgeführten Messungen senkrecht in jeden Modul stellt.

18. Verfahren nach Anspruch 13, dadurch gekennzeichnet, dass man mittels Absaugung die Abfälle sowie die Probegläser, die zur Messungen gedient haben automatisch entleert.
